# EUROPEAN PATENT SPECIFICATION

(11) **EP 2 979 717 B1**
(45) Date of publication and mention of the grant of the patent: **12.08.2020**
(21) Application number: 13879674.3
(22) Date of filing: 25.03.2013
(51) Int. Cl.: A61M 5/34, A61M 5/28, A61M 5/32, A61L 2/00, A61L 9/00

(54) **SYRINGE WITH INJECTION NEEDLE, AND MANUFACTURING METHOD OF SYRINGE WITH INJECTION NEEDLE**
SPRITZE MIT INJEKTIONSNADEL UND HERSTELLUNGSVERFAHREN EINER SPRITZE MIT INJEKTIONSNADEL
SERINGUE AVEC AIGUILLE D'INJECTION ET PROCÉDÉ DE FABRICATION D'UNE SERINGUE AVEC AIGUILLE D'INJECTION

(43) Date of publication of application: 03.02.2016
(73) Proprietor: Terumo Kabushiki Kaisha, Tokyo 151-0072 (JP)
(72) Inventor: TAKEMOTO, Masafumi, Fujinomiya-shi Shizuoka 418-0004 (JP)
(74) Representative: Casalonga
(86) International application number: PCT/JP2013/058542
(87) International publication number: WO 2014/155481

(56) References cited:
- EP-A1- 1 285 672
- WO-A1-2012/003221
- JP-A- H08 206 201
- JP-A- 2012 254 102
- US-A- 2 551 414
- US-A- 3 782 383
- US-A1- 2012 010 573

## Description

### Technical Field

The present invention relates to a syringe with an injection needle in which an injection needle is directly jointed to an outer cylinder.

### Background Art

A syringe with an injection needle has an injection needle preliminarily jointed to a cylinder tip of an outer cylinder made of resin. The syringe with an injection needle is used as a prefilled syringe in which a drug solution is preliminarily contained, and as a microsyringe adopted for small amount administration. Patent Literature 1 discloses a manufacturing method for a syringe with an injection needle in which a metallic injection needle is jointed to an outer cylinder made of resin. The outer cylinder made of resin is molded by injection molding. At this point, the injection needle is fixed inside a cavity of an injection molding die where a cylinder tip portion of the outer cylinder is molded while a joint portion protrudes therebetween. Therefore, molten thermoplastic resin is injected into the cavity, thereby covering a jointed outer peripheral surface of the injection needle with resin. When the resin has cooled and solidified, the resin contracts and stress that fastens the injection needle is generated, thereby jointing the injection needle to the outer cylinder. According to this manufacturing method, use of an adhesion bond is not needed to joint the injection needle to the outer cylinder. Therefore, there is no possibility that the drug solution inside the outer cylinder may be contaminated by the adhesion bond, and a safe syringe having a stable quality can be produced on a large scale.

### Citation List

### Patent Literature

Patent Literature 1: WO 2008/139982 A
Patent Literature 2: US 2012/010573
Patent Literature 3: WO2012/003221
Patent Literature 4: US 2 551 414
Patent Literature 5: EP 1 285 672
Patent Literature 6: US 3 782 383

### Summary of Invention

### Technical Problem

Because medical devices such as a syringe with an injection needle need to be clean, sterilization is applied using an autoclave or ethylene oxide gas after being manufactured. Such sterilization by an autoclave or ethylene oxide gas causes a temperature load. The temperature load increases the flowability of the resin forming the outer cylinder, and relieves the stress that fastens the injection needle. As a result, a joint strength between the injection needle and the outer cylinder is decreased. Therefore, according to the syringe with an injection needle disclosed in Patent Literature 1, the joint strength between the injection needle and the outer cylinder is largely decreased by performing sterilization by an autoclave or ethylene oxide gas, and result in a possibility that the injection needle will be unintentionally released from the outer cylinder or the injection needle will be pushed into the outer cylinder at the time of puncture.

The present invention is made to solve the above-described problem, and directed to providing a syringe with an injection needle in which the injection needle is firmly jointed to an outer cylinder without largely decreasing the joint strength between the injection needle and the outer cylinder even when the flowability of resin forming the outer cylinder is increased when sterilization by an autoclave or ethylene oxide gas is performed and the stress fastening the injection needle is relieved.

### Solution to Problem

A syringe with an injection needle to which the present invention is applied includes: a cylindrical-shaped outer cylinder molded with resin and including a cylinder tip at a distal end, an opening at a proximal end, and a barrel section including a cavity located between the cylinder tip and the opening and configured to be filled with a drug solution; and a metallic injection needle including a joint portion fixed inside the cylinder tip and a needle tip projecting to a distal end side beyond the cylinder tip, and allowing communication between the cavity and an outside of the outer cylinder. The injection needle is fixed inside the cylinder tip by insert molding or heat welding. In the syringe with an injection needle, an uneven portion having an average surface roughness of at least 0.2 µm is formed in a region having an area of at least 1 mm² on an outer peripheral surface of the joint portion, the joint portion is firmly fixed inside the cylinder tip by the resin which closely contacts and covers respective recesses and protrusions of the uneven portion, and sterilization by an autoclave or ethylene oxide gas is applied to the outer cylinder having the injection needle fixed inside the cylinder tip.

In the syringe with an injection needle, preferably, the uneven portion is formed by blast processing.

In the syringe with an injection needle, the uneven portions may be formed with a plurality at an equal interval in a circumferential direction of the injection needle.

In the syringe with an injection needle, preferably, a proximal end portion of the injection needle is not provided with the uneven portion.

In syringe with an injection needle, preferably, a length of the joint portion in an axial direction of the injection needle is a maximum of 10 mm.

The syringe with an injection needle may include a cap detachably attached to the cylinder tip and configured to seal the needle tip.

Preferably, the syringe with an injection needle includes a drug solution filled in the cavity, and a gasket slidably disposed in the barrel section in a liquid-tight manner.

Provided is a manufacturing method for a syringe with an injection needle which includes: a cylindrical-shaped outer cylinder molded with a resin and including a cylinder tip at a distal end, an opening at a proximal end, and a barrel section including a cavity located between the cylinder tip and the opening and configured to be filled with a drug solution; and a metallic injection needle including a joint portion fixed inside the cylinder tip and a needle tip projecting to a distal end side beyond the cylinder tip, and allowing communication between the cavity and an outside of the outer cylinder. The manufacturing method includes: forming an uneven portion having an average surface roughness of at least 0. 2 µm in a region having an area of at least 1 mm² on an outer peripheral surface of the joint portion; molding the outer cylinder with the injection needle by performing insert molding at the time of molding the outer cylinder with the resin, such that the joint portion is firmly fixed inside the cylinder tip by virtue of the resin which closely contacts and covers respective recesses and protrusions of the uneven portion, or thermally welding the injection needle to the outer cylinder after molding the outer cylinder with the resin, such that the joint portion is firmly fixed inside the cylinder tip by the resin which closely contacts and covers respective recesses and protrusions of the uneven portion; and sterilizing the outer cylinder using an autoclave or ethylene oxide gas.

In the manufacturing method for a syringe with an injection needle, preferably, forming the uneven portion is performed by blast processing.

In the manufacturing method for a syringe with an injection needle, preferably, the blast processing is executed by shooting blasting material from a plurality of directions at an equal interval in a circumferential direction of the injection needle .

### Advantageous Effects of Invention

According to the syringe with an injection needle of the present invention, the uneven portion having an average surface roughness of at least 0.2 µm is formed in the region having the area of at least 1 mm² on the outer peripheral surface of the joint portion of the injection needle. The respective recesses and protrusions of the uneven portion are closely contacted and covered by the resin of the outer cylinder by virtue of the resin of the outer cylinder flowing into the recesses. This exhibits a strong anchor effect between the injection needle and the outer cylinder. With the anchor effect, the joint strength between the outer cylinder and the injection needle is enhanced. As a result, even when the stress that fastens the injection needle jointed to the cylinder tip is relieved by sterilization using an autoclave or ethylene oxide gas applied to the outer cylinder, the joint strength between the injection needle and the outer cylinder is not decreased in this syringe with an injection needle because of the strong anchor effect overcoming such a situation.

### Brief Description of Drawings

Fig . 1 is a cross-sectional view illustrating an embodiment of a syringe with an injection needle to which the present invention is applied.
Fig. 2 is a schematic enlarged partial cross-sectional view illustrating an uneven portion included in an injection needle used for the syringe with an injection needle to which the present invention is applied.
Figs. 3 (a) to 3(f) are front views illustrating different examples of the injection needle used for the syringe with an injection needle to which the present invention is applied.
Fig. 4 is a graph illustrating a correlation between an average surface roughness of unevenness formed on an injection needle of the syringe with the injection needle and joint strength of a injection needle.

### Description of Embodiments

In the following, embodiments of the present invention will be described in detail, but the scope of the present invention is not limited to the embodiments.

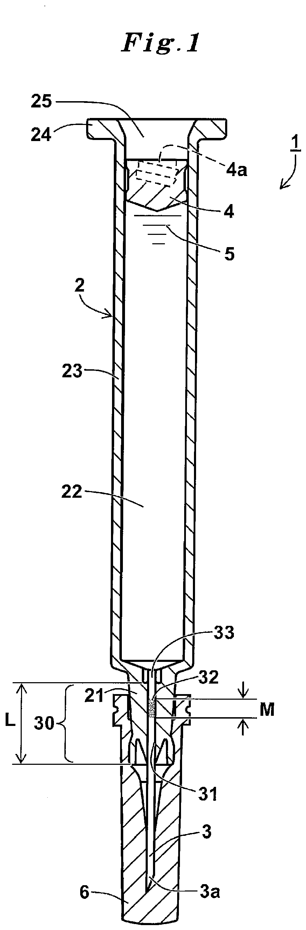

Fig. 1 is a cross-sectional view illustrating an embodiment of a syringe with an injection needle according to the present invention. A syringe with an injection needle 1 is a prefilled syringe that includes an outer cylinder 2 made of resin, a metallic injection needle 3 fixed to a cylinder tip 21 thereof, a gasket 4 inserted into a cavity 22 of the outer cylinder 2, a drug solution 5 filled in the cavity 22 located between the cylinder tip 21 and the gasket 4, and a cap 6 that seals a needle tip 3a of the injection needle 3.

The outer cylinder 2 includes the cylinder tip 21 formed at a distal end of a barrel section 23, an opening 25 at a proximal end of the barrel section 23, and a flange 24 formed at a periphery thereof. The cylinder tip 21 has a more reduced diameter than the barrel section 23, and the needle tip 3a of the injection needle 3 projects from the distal end. The injection needle 3 has a thickness of 34 to 25 G (outer diameter of about 0.18 to 0.53 mm) .

The injection needle 3 penetrates the cylinder tip 21 and allows communication between the inside and the outside of the outer cylinder 2. The injection needle 3 is fixed by being directly jointed to the outer cylinder 2. Ajointed point between the injection needle 3 and the outer cylinder 2 is a joint portion 30. An outer peripheral surface of the joint portion 30 is a jointed outer peripheral surface 31, which is closely contacted and covered by the resin with which the outer cylinder 2 is molded. The injection needle 3 includes a fine uneven portion 32 at a part in substantially a middle of the jointed outer peripheral surface 31.

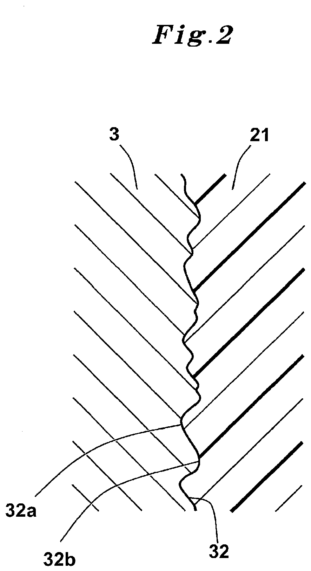

Fig. 2 is a schematic enlarged partial cross-sectional view illustrating the uneven portion 32. The uneven portion 32 is formed with recesses 32a and protrusions 32b. The uneven portion 32 is a rough surface formed by blast processing, and is formed surrounding a part of the jointed outer peripheral surface 31 in a certain range having a width M. The uneven portion 32 exhibits an anchor effect between the injection needle 3 and the outer cylinder 2 by virtue of the fact that the resin forming the outer cylinder 2 closely contacts and covers the respective recesses 32a and the respective protrusions 32b. Because of this anchor effect, joint strength between the injection needle 3 and the outer cylinder 2 is significantly more enhanced than the case where the uneven portion 32 is not formed. Particularly, the uneven portion 32 is preferably formed in a region having an area of 1 mm² or more on the outer peripheral surface of the joint portion 30 (jointed outer peripheral surface 31) so as to have an average surface roughness of 0.2 µm or more.

When the outer cylinder 2 is molded by injection molding, molten resin flows inside a cavity of a die and flows into the recesses 32a of the uneven portion 32. When the resin is cooled and solidified in this state, the resin is caught in the respective recesses 32a and respective protrusions 32b, thereby exhibiting the anchor effect. Particularly, when the uneven portion 32 having the average surface roughness of 0.2 µm or more is formed on the region having the area of 1 mm² or more on the jointed outer peripheral surface 31, the injection needle 3 and the outer cylinder 2 are engaged via the respective recesses 32a and respective protrusions 32b. As a result, a stronger anchor effect can be obtained therebetween. Even when stress that fastens the injection needle 3 is relieved under a temperature load caused by high-pressure steam sterilization by an autoclave or sterilization by ethylene oxide gas applied to the syringe with an injection needle 1, the joint strength between the injection needle 3 and the outer cylinder 2 is not largely decreased because of this strong anchor effect. Therefore, the injection needle 3 is prevented from being pushed into the outer cylinder 2 when the injection needle 3 is punctured into skin, and the injection needle 3 is prevented from being released from the outer cylinder 2 when the injection needle 3 is pulled out from the skin.

The joint strength between the injection needle 3 and the outer cylinder 2 is correlated with the average surface roughness of the uneven portion 32. The larger the average surface roughness of the uneven portion 32 is, the larger a height difference between the recess 32a and the protrusion 32b is. This increases the anchor effect, and the joint strength between the injection needle 3 and the outer cylinder 2 is enhanced. The average surface roughness of the uneven portion 32 is needed to be at least 0.2 µm in order to keep sufficient joint strength between the injection needle 3 and the outer cylinder 2 at the time of detaching the cap 6 or puncturing/pulling the injection needle 3 even when sterilization using the autoclave or ethylene oxide gas is applied to the outer cylinder 2. Note that, preferably, the average surface roughness of the uneven portion 32 is half or less than a thickness of the injection needle 3 at a maximum. This prevents deformation at an inner peripheral surface of the injection needle 3 at the time of forming the uneven portion 32 on the jointed outer peripheral surface 31 of the joint portion 30.

Further, the joint strength between the injection needle 3 and the outer cylinder 2 is correlated with the area of the jointed outer peripheral surface 31 where the uneven portion 32 is formed. The larger the area of the joint portion 30 where the uneven portion 32 is formed is, the larger the number of the recesses 32a and protrusions 32b is. As a result, the anchor effect is increased, and the j oint strength between the injection needle 3 and the outer cylinder 2 is enhanced. The joint strength must be sufficient and not largely decreased even when sterilization using the autoclave or ethylene oxide gas is applied to the outer cylinder 2. Therefore, the area of the region where the uneven portion 32 is formed on the jointed outer peripheral surface 31 of the joint portion 30 is needed to be at least 1 mm² in order to achieve a sufficient joint strength between the outer cylinder 2 and the injection needle 3. The region may be an entire surface of the jointed outer peripheral surface 31 of the joint portion 30 at a maximum.

For example, in the case where the thickness of the injection needle 3 is 34 G (outer diameter of about 0.18 mm), the width M of the uneven portion 32 is needed to be at least 1.8 mm such that the area of the region where the uneven portion 32 is formed is 1 mm² or more. When the area of the region where the uneven portion 32 is formed is smaller than 1 mm², the anchor effect becomes insufficient.

In the case of sterilization by an autoclave, a temperature load of 121 to 125°C is applied, and in the case of sterilization by ethylene oxide gas, a temperature load of 40 to 70 °C is applied to the syringe with an injection needle 1. Even when the resin of the outer cylinder 2 is rather softened by the temperature load and the stress fastening the injection needle 3 is relieved, the resin of the outer cylinder 2 is kept engaged and caught at the uneven portion 32 by the uneven portion 32 having the above-mentioned sufficient area and average surface roughness. Therefore, according to the syringe with an injection needle 1, a sufficient joint strength is kept between the injection needle 3 and the outer cylinder 2 before and after sterilization or during sterilization. As a result, there is no possibility that the injection needle 3 easily falls off from the outer cylinder 2.

Further, the jointed outer peripheral surface 31 exhibits the sufficient anchor effect at the uneven portion 32. As a result, there is no need to specially keep a wide area for the jointed outer peripheral surface 31 in order to achieve a sufficient joint strength between the injection needle 3 and the outer cylinder 2 even after sterilization by an autoclave or ethylene oxide gas. Therefore, a length L of the jointportion 30 in an axial direction of the injection needle 3 can be shortened. As a result, a length of the cylinder tip 21 can be shortened along with this. More specifically, the length L of the joint portion 30 in the axial direction of the injection needle 3 may be set to 10 mm or less. Thus, since the injection needle 3 includes the uneven portion 32, the syringe with an injection needle 1 can be downsized by shortening the injection needle 3 and the cylinder tip 21. This can reduce weight of using material and can simplify the injection molding die, thereby achieving cost reduction and inexpensive manufacturing. Further, the drug solution which remains inside the injection needle 3 and is wasted can be reduced to an extremely small quantity.

The gasket 4 is an elastic body such as rubber and elastomer, which closely contacts an inner wall of the barrel section 23 in an airtight manner and is slidably disposed in the barrel section 23. The gasket 4 has a fitting hole 4a at the proximal end. A plunger (not illustrated) is screwed and fitted into the fitting hole 4a. The plunger is adopted to push the gasket 4 and slide the same toward the cylinder tip 21. Along with the sliding operation, the gasket 4 discharges the drug solution 5 from the needle tip 3a/injection needle 3.

The cap 6 is an elastic body such as rubber and elastomer, and covers the cylinder tip 21 and the injection needle 3 when the needle tip 3a of the injection needle 3 is straightly punctured into the cap 6. The cap 6 prevents leakage of the drug solution 5 contained in the cavity 22 from the needle tip 3a while protecting the injection needle 3 from being bent and contaminated. Since the injection needle 3 is punctured into the cap 6, the cap 6 fastens an outer peripheral surface of the injection needle 3 on the distal end side. When the cap 6 is pulled out and detached, the injection needle 3 is also pulled out together with the cap 6 because of the fastening force. However, since the uneven portion 32 is formed and the anchor effect is exhibited, the joint strength is enhanced. Therefore, the injection needle 3 is prevented from being slipped out of the outer cylinder 2.

The drug solution 5 is contained in the cavity 22 while being interposed between the cylinder tip 21 and the gasket 4. The gasket 4 inserted into the barrel section 23 in a liquid-tight manner, and the cap 6 that seals the needle tip 3a prevents the drug solution 5 from being contaminated leaking when the syringe with an injection needle 1 is transported or stored. Note that some of the drug solution 5 may be stored inside the injection needle 3.

The syringe with an injection needle 1 is manufactured as follows. First, a process of forming the uneven portion 32 is performed by blast processing in the region having an area of 1 mm² or more on the jointed outer peripheral surface 31 of the injection needle 3. According to the blast processing, fine unevenness can be easily formed by means of blasting material and blasting conditions . During the blast processing, a portion of the joint portion 30 of the injection needle 3, where the uneven portion 32 is not formed, is protected by applying masking with a rubber sheet and the like. After the injection needle 3 is inserted into a blasting machine, the blasting material is shot from a nozzle end to an exposed portion for a predetermined period while being rotated about the axis of the injection needle 3. Thus, the uneven portion 32 is formed. As the blasting material, a metal-based material, such as cast iron, aluminum, and zinc, a ceramic-based material, such as alumina, silicon carbide, and silica, a resin-based material, such as nylon and polycarbonate, dry ice, and glass beads may be selected. A suitable average particle diameter of the blasting material is 20 to 250 µm. Further, injection air pressure to shoot the blasting material is 0.1 to 10 MPa, and a shooting period is 2 to 30 seconds. For example, in the case where the injection needle 3 is made of stainless steel and has a thickness of 27 G, silica alumina having an average particle diameter of 70 µm is selected as the blasting material, and the uneven portion 32 is formed by performing the processing under an injection air pressure of about 3.5 MPa for 10 seconds, thereby achieving an average surface roughness of about 2 µm.

Further, the uneven portion 32 having an average surface roughness of 0.2 µm may be achieved by performing the blast processing, using blasting material having a particularly small average particle diameter. Because the uneven portion 32 having an average surface roughness of 0.2 µm includes the far more number of recesses and protrusions, compared to an uneven portion having an average surface roughness of several µm, a sufficient anchor effect can be obtained. Due to this, a sufficient joint strength can be obtained between the injection needle 3 and the outer cylinder 2.

Next, a process in which the injection needle 3 is jointed to the outer cylinder 2 by applying an insert molding to the injection molding of the outer cylinder 2 is performed. At this point, the outer cylinder 2 is molded by using an injection molding die of a female die to form the outer shape of the outer cylinder 2 and a core pin which is a male die to form the cavity 22 of the outer cylinder. First, the injection needle 3 formed with the uneven portion 32 is fixed to the injection molding die. At this point, the uneven portion 32 is set inside a cavity of the female die that molds the cylinder tip 21. Further, the injection needle 3 is fixed by inserting a proximal end portion 33 of the injection needle 3 into a holding hole provided at a tip of the core pin. Next, a medical thermoplastic resin pellet used for molding the outer cylinder 2 is put into the injection molding machine and melted at a high temperature. The outer cylinder 2 is molded by injecting the molten resin into the cavity of the die under high pressure . The molten resin closely contacts and covers the respective recesses 32a and respective protrusions 32b of the uneven portion 32 of the injection needle 3 set inside the cavity, and then is cooled and solidified. This exhibits the anchor effect between the injection needle 3 and the outer cylinder 2, and a strong joint strength can be obtained. Note that, preferably, the uneven portion 32 is not formed on the outer peripheral surface of the proximal end portion 33 by applying masking thereon. With this structure, the proximal end of the injection needle 3 can be surely held at the holding hole of the core pin of the injection molding die.

The injection needle 3 is straightly punctured into the cap 6 having a recess fittable with the cylinder tip 21, and then the cap 6 is attached to the cylinder tip 21. The syringe in this state is hung up and stored inside an outer cylinder storage container which can store a plurality of these syringes (not illustrated). A protection film is adhered to an opening of the outer cylinder storage container to seal the opening. In a state that the syringe with an injection needle 1 is stored in the outer cylinder storage container, sterilization by an autoclave or ethylene oxide gas is applied. Then, the syringe with an injection needle is packed together with the outer cylinder storage container and transported to a fabricating lab where the drug solution is filled.

Inside a clean room of the fabricating lab where the drug solution is filled, the protection film of the outer cylinder storage container is peeled off, and the outer cylinder storage container is placed at a predetermined position of a chemical filling device (not illustrated) . A drug solution supply nozzle is inserted into the opening 25, and a predetermined amount of the drug solution 5 is filled into the cavity 22. Next, the drug solution 5 is contained in the cavity 22 by inserting the gasket 4 from the opening 25. Thus, the syringe with an injection needle 1 which is a prefilled syringe is manufactured.

Usage of the syringe with an injection needle 1 is as follows . The syringe with an injection needle 1 is taken out from the outer cylinder storage container, and put into a package bag where it is individually sterilized and sealed, and then shipped out. The syringe with an injection needle 1 is transported to a hospital and the like, and taken out from the package bag immediately before the drug solution 5 is administered to a patient. The plunger (not illustrated) is screwed and fitted into the fitting hole 4a of the gasket 4, and the cap 6 is removed. When the injection needle 3 is punctured into the patient's skin and the proximal end of the plunger is pushed, the drug solution 5 is pushed out by the gasket 4 and discharged from the syringe with an injection needle 1, and administered to the patient. Note that the syringe with an injection needle 1 may be shipped out with the plunger fitted thereto. After that, the syringe with an injection needle 1 is dumped as infectious medical waste together with the gasket 4 and the plunger in accordance with a predetermined procedure.

Besides the above-described prefilled syringe, the syringe with an injection needle 1 may be a pre-fillable syringe which includes only an outer cylinder 2 and an injection needle 3 and is capable of being filled with the drug solution 5, or a syringe formed by attaching the cap 6 to this pre-fillable syringe.

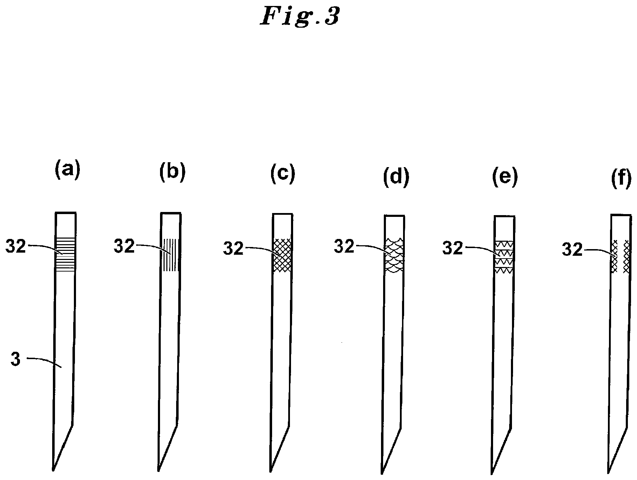

In Fig. 1, the example of forming the uneven portion 32 by the blast processing has been described, but what is necessary is that the uneven portion 32 is formed such that the resin forming the cylinder tip 21 flows into the recesses 32a and is caught by the respective recesses 32a and respective protrusions 32b. In Fig. 1, the example of forming the uneven portion 32 of the rough surface by the blast processing was described, but besides that, the uneven portion 32 may be formed of, for example, a single-cut file illustrated in Fig. 3(a), a single-cut file of vertical stripes illustrated in Fig. 3(b), a double-cut file illustrated in Fig. 3(c), a vixen file illustrated in Fig. 3(d), or a rasp-cut file illustrated in Fig. 3(e). Further, as illustrated in Fig. 3(f), a plurality of uneven portion 32 may be formed at an equal interval in a circumferential direction of the injection needle 3. These may be formed by plastic forming in which the uneven portion 32 is shaped by pressing and transferring the file shape. In other examples, the uneven portion 32 maybe formed by a grinding process in which the recesses 32a are formed by grinding, and an etching process in which the recesses 32a are formed by applying an etching agent, an ion beam, and so on.

Meanwhile, among these processes, the uneven portion 32 is preferably formed by the blast processing. In the case of the blast processing, the uneven portion 32 can be formed without damaging the needle tip 3a. Further, also according to the blast processing, the inner peripheral surface of the injection needle 3 is not deformed at the time of forming the uneven portion 32. Therefore, injection resistance is not increased when the drug solution 5 is administered. Moreover, in the blast processing, a chemical agent that may affect the drug solution 5 is not used. Therefore, the remaining drug solution 5 is not contaminated by such a chemical agent.

In Fig. 1, an example of rotating the injection needle 3 at the time of blast processing was described, but the blasting material may be shot from a plurality of directions at an equal interval in the circumferential direction thereof without rotating the injection needle 3. In this manner, the plurality of uneven portions 32 may be formed at the equal interval in the circumferential direction of the injection needle 3 as illustrated in Fig. 3(f). With this structure, stress is not concentrated on only one side in the circumferential direction of the injection needle 3 even in the case where a force due to pull or push is applied against the injection needle 3 in the axial direction of the injection needle 3. Therefore, the joint between the joint portion 30 of the injection needle 3 and the cylinder tip 21 of the outer cylinder 2 is prevented from being fractured. In the case of continuously forming the uneven portion 32 in the circumferential direction of the injection needle 3 illustrated in Fig. 1, numerous recesses 32a and protrusions 32b are also formed in the injection needle 3 in the circumferential direction in a range having the width M same as the case where the plurality of uneven portions 32 is formed at an equal interval in the circumferential direction of the injection needle 3,.

An example of forming the uneven portion 32 at a part of the jointed outer peripheral surface 31 of the injection needle 3 has been described, but the uneven portion 32 may be formed at an entire portion of the jointed outer peripheral surface 31 as well.

In Fig. 1, the jointing process is performed by applying an insert molding to the injection molding of the outer cylinder 2 was described as the process of jointing the injection needle 3 to the outer cylinder 2. However, the injection needle 3 may be jointed to the outer cylinder 2 by a process of heat welding as well. The outer cylinder 2 is preliminarily molded by injection molding or the like. The cylinder tip 21 includes an opening at the distal end and the opening, having a diameter slightly larger than the outer diameter of the injection needle 3, penetrates the outer cylinder 2, thereby allowing communication between the inside and the outside of the outer cylinder 2. The injection needle 3 is inserted from the proximal end thereof into the opening of the cylinder tip 21 up to a predetermined position where the entire uneven portion 32 faces an inner wall surface of the cylinder tip 21. Next, the inner wall surface of the cylinder tip 21 facing the outer peripheral surface of the injection needle 3 is irradiated with a laser or high-frequency radiation to melt the inner wall surface of the cylinder tip 21, thereby jointing the injection needle 3 to the outer cylinder 2. With this heat welding, there is no need to use a die having a structure to fix the injection needle 3. Therefore, the syringe with an injection needle 1 can be manufactured at a low cost. Note that a resin jointing member that is cylindrically shaped (not illustrated) may be disposed between the cylinder tip 21 and the injection needle 3 in order to enhance liquid-tightness between the cylinder tip 21 and the injection needle 3. In this case, the injection needle 3 is inserted into the jointing member, and is thermally welded while being pressed into the cylinder tip 21.

The material of the outer cylinder 2 is selected in view of chemical resistance, heat resistance, gas/bacteria barrier properties, safety to living bodies, transparency, and so on. For example, polyolefin resin, such as polyethylene, polypropylene, and cyclic polyolefin; polystyrene; polycarbonate; polyester, such as polyethylene terephthalate; and polyamide may be used. Especially, it is preferable to use cyclic olefin homopolymer or cyclic olefin copolymer as the material, which is a resin that is transparent such that the drug solution contained inside can be visually confirmed from the outside and that has little interaction with the drug solution. Such material is formed by molding. As the molding method, an injection molding method, a blow molding method, a heat molding method, or the like, may be used, and among these methods, an injection molding method is preferable.

The material of the injection needle 3 is material that can be used in the insert molding or heat welding, and is selected in the view of chemical resistance, heat resistance, gas/bacteria barrier properties, safety to living bodies, and so on. For example, stainless steel and nickel-free stainless steel may be selected, but stainless steel is preferable because it may be easily molded at a low cost.

The materials of the cap 6 and gasket 4 are selected in the view of chemical resistance, heat resistance, gas/bacteria barrier properties, safety to living bodies, and airtightness. For example, a olefin-based, polyurethane-based, polyester-based, polyamide-based, or styrene-based thermoplastic elastomer, or a rubber material, such as natural rubber, isoprene rubber, butadiene rubber, styrene-butadiene rubber, and silicone rubber, may be used.

In the case where the syringe with an injection needle 1 constitutes a prefilled syringe, for example, a bio-pharmaceutical, such as vaccines against influenza, tetanus, streptococcus pneumoniae, poliomyelitis, Japanese encephalitis, rubella, measles, yellow fever, Hib, hepatitis, chickenpox, rabies, rotavirus, the mumps, prevention of cervical cancer, MQ, DT, DPT, etc., are listed as a drug solution 5 contained inside the outer cylinder 2. Besides vaccines, for example, insulin preparations, sugar injection solution like glucose, injection solution for correcting electrolyte such as sodium chloride or potassium lactate, contrast agents, steroid preparations, protein decomposition enzyme inhibitors, lipid microsphere, antibiotics, anticancer drug, heparin calcium, anesthetics, antibody drug, etc. may be listed.

### [Example]

In the following, a description will be given for an example of experimentally manufacturing a syringe with an injection needle to which the present invention is applied.

### (Example)

A stainless steel injection needle (27 G) having an outer diameter of 0.41 mm, an inner diameter of 0.28 mm, and a length of 22 mm was used as samples of an example to which the present invention is applied. The uneven portion 32 was formed by applying a sandblast processing to an outer peripheral surface in a region having a length up to 9 mm from the proximal end of the injection needle 3 in the axial direction of the injection needle 3. Next, an average surface roughness Ra was acquired by measuring the surface roughness of the uneven portion 32 having the length of 9 mm in the axial direction of the injection needle 3, using a surface roughness gauge (Olympus, Product Name OLS4000) . After cleaning the injection needle 3, the injection needle 3 was fixed to an injection molding die. A pellet made of cyclic polyolefin resin was put into an injection molding machine and melted at a high temperature, and then the molten resin was injected into the cavity of the die under high pressure, thereby manufacturing the syringe with an injection needle 1 in which the injection needle 3 was jointed and fixed to the outer cylinder 2. Note that the proximal end portion 33 of the injection needle 3 was not included in the joint portion 30 because the proximal end portion 33 was held by the core pin while projecting outside the cavity of the female die by 1 mm. Therefore, the joint portion 30 which joints the injection needle 3 to the outer cylinder 2 had a length L in the axial direction of the injection needle 3 of 8 mm which was shorter than the width M in the axial direction where the uneven portion 32 was formed. More specifically, the area of the jointed outer peripheral surface 31 where the uneven portion 32 was formed was 10.3 mm².

### (Comparative Example)

As a comparative example, in which the present invention is not applied, an injection needle in which the sandblast processing was not applied was prepared. The only difference from the example was that the sandblast processing was not applied. Other aspects remained the same as the example, and the syringe with an injection needle was manufactured by injection molding after measuring the surface roughness and acquiring the average surface roughness.

### (Tensile Test)

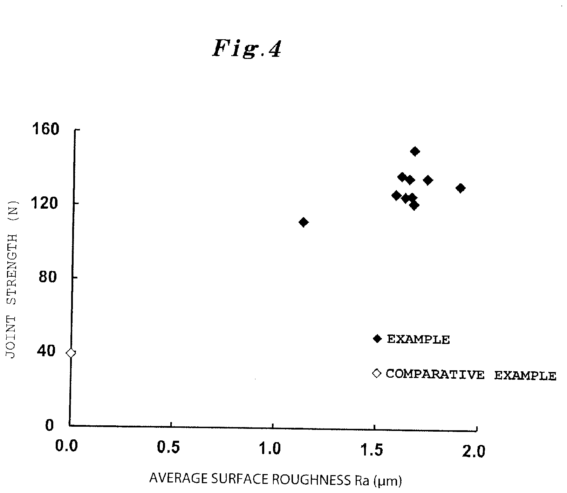

Tensile tests were carried out by using a tensile testing machine (Shimadzu Corporation, Product Name AGS-H) for the samples of the example and the comparative example. The syringe with an injection needle was fixed to the tensile testing machine such that the flange of the outer cylinder was positioned on the lower side and the needle tip was positioned on the upper side, and the distal end side of the injection needle was held by an air chuck. Then, the injection needle was pulled at a speed of 50 mm/min until the injection needle was pulled out, and a maximum resistance value at this point was acquired as the joint strength. Fig. 4 is a graph in which the average surface roughness Ra (µm) is plotted on a horizontal axis and the joint strength (N) is plotted on a vertical axis. The joint strength of the samples of the example resulted in that: a sample having an Ra of about 1 µm had the joint strength of about 110 N and a sample having an Ra of 1.5 to 2.0 µm had the joint strength of about 120 to 150 N. In contrast, the sample of the comparative example having an Ra of 0 µm had a joint strength of about 40 N. The syringe with an injection needle 1 in the example applied with the sandblast processing and formed with the uneven portion 32 had a joint strength higher than the comparative example that did not have this processing applied.

### (Sterilization by Autoclave)

Sterilization to the samples of the example and the comparative example was performed by an autoclave at temperature 121°C for a period of 20 minutes. After that, the tensile tests were carried out and comparison was made between a sample of the example and a sample of the comparative example. The results are shown in Table 1. In Table 1, the joint strength is rated on three scales: 80 N or higher is indicated by O, 40 to 80N by Δ, and 40 N by ×.

**[Table 1]**

| | Sterilization by Autoclave | |
|---|---|---|
| | Not Applied | Applied |
| Example (provided with uneven portion) | ○ | ○ |
| Comparative Example (without uneven portion) | Δ | × |

As is obvious from TABLE 1, the syringe with an injection needle in the example in which the injection needle includes an uneven portion having an average surface roughness of 0.2 µm or more keeps a high joint strength before and after sterilization by the autoclave. On the other hand, the syringe with an injection needle in the comparative example, which was not provided with an uneven portion showed a joint strength that was insufficient even before the sterilization by the autoclave and rapidly decreased after sterilization. Thus, the difference between the example and the comparative example was noticeable.

### Industrial Applicability

A syringe with an injection needle according to the present invention may be used as a prefilled syringe in which a drug solution is preliminarily contained inside an outer cylinder after performing sterilization by an autoclave or ethylene oxide gas, or as a syringe to suction injection solution at the time of use, and is used to administer the drug solution to a patient or the like by an injection.

### Reference Signs List

- 1:: Syringe with injection needle
- 2:: Outer cylinder
- 3:: Injection needle
- 3a:: Needle tip
- 4:: Gasket
- 4a:: Fitting hole
- 5:: Drug solution
- 6:: Cap
- 21:: Cylinder tip
- 22:: Cavity
- 23:: Barrel section
- 24:: Flange
- 25:: Opening
- 30:: Joint portion
- 31:: Jointed outer peripheral surface
- 32:: Uneven portion
- 32a:: Recess
- 32b:: Protrusion
- 33:: Proximal end portion
- L:: Length
- M:: Width

## Claims

1. A syringe (1) with an injection needle (3), comprising:
a cylindrical-shaped outer cylinder (2) molded with a resin and including a cylinder tip (21) at a distal end, an opening (25) at a proximal end, and a barrel section (23) including a cavity (22) located between the cylinder tip (21) and the opening (25) and configured to be filled with a drug solution (5); and
a metallic injection needle (3) including a joint portion (30) fixed inside the cylinder tip (21) and a needle tip (3a) projecting to a distal end side beyond the cylinder tip (21), and allowing communication between the cavity (22) and an outside of the outer cylinder (2), the injection needle (3) being fixed inside the cylinder tip (21) by insert molding or heat welding,
**characterized in that**
an uneven portion (32) having an average surface roughness of at least 0.2 µm is formed in a region having an area of at least 1 mm² on an outer peripheral surface of the joint portion (30),
the joint portion (30) is firmly fixed inside the cylinder tip (21) by the resin which closely contacts and covers respective recesses (32a) and protrusions (32b) of the uneven portion (32),
the outer cylinder (2) having the injection needle (3) fixed inside the cylinder tip (21) has been sterilized by an autoclave or ethylene oxide gas,
and
the injection needle (3) includes a proximal end portion (33) that is disposed opposite to the needle tip (3a) of the injection needle (3) and is not provided with the uneven portion (32).

2. The syringe (1) with an injection needle (3) according to claim 1, wherein the uneven portion (32) has been formed by blast processing.

3. The syringe (1) with an injection needle (3) according to claim 1, wherein the uneven portion (32) is formed with a plurality of uneven portions (32) at an equal interval in a circumferential direction of the injection needle (3).

4. The syringe (1) with an injection needle (3) according to claim 1, wherein a length of the joint portion (30) in an axial direction of the injection needle (3) is a maximum of 10 mm.

5. The syringe (1) with an injection needle (3) according to claim 1, comprising a cap (6) detachably attached to the cylinder tip (21) and configured to seal the needle tip (3a).

6. The syringe (1) with an injection needle (3) according to claim 5, comprising a drug solution (5) filled in the cavity (22), and a gasket (4) slidably disposed in the barrel section (23) in a liquid-tight manner.

7. A manufacturing method for a syringe (1) with an injection needle (3) having:
a cylindrical-shaped outer cylinder (2) molded with a resin and including a cylinder tip (21) at a distal end, an opening (25) at a proximal end, and a barrel section (23) including a cavity (22) located between the cylinder tip (21) and the opening (25) and configured to be filled with a drug solution (5); and
a metallic injection needle (3) including a joint portion (30) fixed inside the cylinder tip (21) and a needle tip (3a) projecting to a distal end side beyond the cylinder tip (21), and allowing communication between the cavity (22) and an outside of the outer cylinder (2),
**characterized in that**
the manufacturing method comprises:
forming an uneven portion (32) having an average surface roughness of at least 0.2 (µm in a region having an area of at least 1 mm² on an outer peripheral surface of the joint portion (30);
molding the outer cylinder (2) with the injection needle (3) by performing insert molding at the time of molding the outer cylinder (2) with the resin, such that the joint portion (30) is firmly fixed inside the cylinder tip (21) by virtue of the resin which closely contacts and covers respective recesses (32a) and protrusions (32b) of the uneven portion (32), or thermally welding the injection needle (3) to the outer cylinder (2) after molding the outer cylinder (2) with the resin, such that the joint portion (30) is firmly fixed inside the cylinder tip (21) by the resin which closely contacts and covers respective recesses (32a) and protrusions (32b) of the uneven portion (32); and
sterilizing the outer cylinder (2) using an autoclave or ethylene oxide gas,
wherein
the injection needle (3) includes a proximal end portion (33) that is disposed opposite to the needle tip (3a) of the injection needle (3) and is not provided with the uneven portion (32).

8. The manufacturing method for a syringe (1) with an injection needle (3) according to claim 7, wherein forming the uneven portion (32) is performed by blast processing.

9. The manufacturing method for a syringe (1) with an injection needle (3) according to claim 8, wherein the blast processing is executed by shooting blasting material from a plurality of directions at an equal interval in a circumferential direction of the injection needle (3).

## Patentansprüche

1. Spritze (1) mit einer Injektionsnadel (3), umfassend:
einen zylindrisch geformten äußeren Zylinder (2), der mit einem Harz geformt ist und eine Zylinderspitze (21) an einem distalen Ende, eine Öffnung (25) an einem proximalen Ende und einen Zylinderabschnitt (23) mit einem Hohlraum (22) aufweist, welcher zwischen der Zylinderspitze (21) und der Öffnung (25) angeordnet ist, und so konfiguriert ist, dass er mit einer Arzneimittellösung (5) gefüllt werden kann; und
eine metallische Injektionsnadel (3) mit einem Verbindungsabschnitt (30), der innerhalb der Zylinderspitze (21) befestigt ist, und einer Nadelspitze (3a), die an einer distalen Endseite über die Zylinderspitze (21) hinaus vorsteht und eine Verbindung zwischen dem Hohlraum (22) und einer Außenseite des äußeren Zylinders (2) ermöglicht, wobei die Injektionsnadel (3) innerhalb der Zylinderspitze (21) durch Einspritzgießen oder Wärmeschweißen befestigt ist,
**dadurch gekennzeichnet, dass**
ein unebener Abschnitt (32), der eine durchschnittliche Oberflächenrauigkeit von mindestens 0,2 µm aufweist, in einem Bereich mit einer Fläche von mindestens 1 mm² auf einer äußeren Umfangsfläche des Verbindungsabschnitts (30) ausgebildet ist,
der Verbindungsabschnitt (30) innerhalb der Zylinderspitze (21) durch das Harz fest fixiert ist, welches die jeweiligen Vertiefungen (32a) und Vorsprünge (32b) des unebenen Abschnitts (32) eng berührt und bedeckt,
der äußere Zylinder (2), der die Injektionsnadel (3) innerhalb der Zylinderspitze (21) befestigt aufweist, durch einen Autoklaven oder durch Ethylenoxidgas sterilisiert worden ist,
und
die Injektionsnadel (3) einen proximalen Endabschnitt (33) aufweist, der gegenüber der Nadelspitze (3a) der Injektionsnadel (3) angeordnet ist und nicht mit dem unebenen Abschnitt (32) versehen ist.

2. Spritze (1) mit einer Injektionsnadel (3) nach Anspruch 1, wobei der unebene Abschnitt (32) durch Strahlbearbeitung gebildet worden ist.

3. Spritze (1) mit einer Injektionsnadel (3) nach Anspruch 1, wobei der unebene Abschnitt (32) mit einer Vielzahl von unebenen Abschnitten (32) in einem gleichen Abstand in einer Umfangsrichtung der Injektionsnadel (3) ausgebildet ist.

4. Spritze (1) mit einer Injektionsnadel (3) nach Anspruch 1, wobei eine Länge des Verbindungsabschnitts (30) in einer axialen Richtung der Injektionsnadel (3) maximal 10 mm beträgt.

5. Spritze (1) mit einer Injektionsnadel (3) nach Anspruch 1, umfassend eine Kappe (6), die abnehmbar an der Zylinderspitze (21) angebracht und so konfiguriert ist, dass sie die Nadelspitze (3a) abdichtet.

6. Spritze (1) mit einer Injektionsnadel (3) nach Anspruch 5, umfassend eine in den Hohlraum (22) gefüllte Arzneimittellösung (5) und eine Dichtung (4), die in dem Zylinderabschnitt (23) in einer flüssigkeitsdichten Weise verschiebbar angeordnet ist.

7. Herstellungsverfahren für eine Spritze (1) mit einer Injektionsnadel (3), aufweisend:
einen zylindrisch geformten äußeren Zylinder (2), der mit einem Harz geformt ist und eine Zylinderspitze (21) an einem distalen Ende, eine Öffnung (25) an einem proximalen Ende und einen Zylinderabschnitt (23) mit einem Hohlraum (22) aufweist, der zwischen der Zylinderspitze (21) und der Öffnung (25) angeordnet und so konfiguriert ist, dass sie mit einer Arzneimittellösung (5) gefüllt werden kann; und
eine metallische Injektionsnadel (3) mit einem Verbindungsabschnitt (30), der innerhalb der Zylinderspitze (21) befestigt ist, und einer Nadelspitze (3a), die an einer distalen Endseite über die Zylinderspitze (21) hinaus vorsteht und eine Verbindung zwischen dem Hohlraum (22) und einer Außenseite des äußeren Zylinders (2) ermöglicht,
**dadurch gekennzeichnet, dass**
das Herstellungsverfahren umfasst:
Ausbilden eines unebenen Abschnitts (32), der eine durchschnittliche Oberflächenrauigkeit von mindestens 0,2 µm aufweist, in einem Bereich mit einer Fläche von mindestens 1 mm² auf einer äußeren Umfangsfläche des Verbindungsabschnitts (30);
Formen des äußeren Zylinders (2) mit der Injektionsnadel (3) durch Ausführen von Einspritzgießen zum Zeitpunkt des Formens des äußeren Zylinders (2) mit dem Harz derart, dass der Verbindungsabschnitt (30) fest innerhalb der Zylinderspitze (21) aufgrund des Harzes fixiert ist, das die jeweiligen Vertiefungen (32a) und Vorsprünge (32b) des unebenen Abschnitts (32) eng berührt und bedeckt, oder durch thermisches Verschweißen der Injektionsnadel (3) mit dem äußeren Zylinder (2) nach dem Formen des äußeren Zylinders (2) mit dem Harz derart, dass der Verbindungsabschnitt (30) fest innerhalb der Zylinderspitze (21) durch das Harz fixiert ist, das die jeweiligen Vertiefungen (32a) und Vorsprünge (32b) des unebenen Abschnitts (32) eng berührt und bedeckt; und
Sterilisieren des äußeren Zylinders (2) unter Verwendung eines Autoklaven oder eines Ethylenoxidgases, wobei
die Injektionsnadel (3) einen proximalen Endabschnitt (33) aufweist, der gegenüber der Nadelspitze (3a) der Injektionsnadel (3) angeordnet ist und nicht mit dem unebenen Abschnitt (32) versehen ist.

8. Herstellungsverfahren für eine Spritze (1) mit einer Injektionsnadel (3) nach Anspruch 7, wobei die Bildung des unebenen Abschnitts (32) durch Strahlbearbeitung durchgeführt worden ist.

9. Herstellungsverfahren für eine Spritze (1) mit einer Injektionsnadel (3) nach Anspruch 8, wobei die Strahlbearbeitung durch Schießen von Strahlmittel aus einer Vielzahl von Richtungen in einem gleichen Abstand in einer Umfangsrichtung der Injektionsnadel (3) durchgeführt wird.

## Revendications

1. Seringue (1) avec une aiguille d'injection (3), comprenant :
un cylindre extérieur de forme cylindrique (2) moulé avec une résine et comportant une pointe cylindrique (21) au niveau d'une extrémité distale, une ouverture (25) au niveau d'une extrémité proximale, et une section corps (23) comportant une cavité (22) située entre la pointe de cylindre (21) et l'ouverture (25) et configurée pour être remplie avec une solution de médicament (5) ; et
une aiguille d'injection métallique (3) incluant une partie articulation (30) fixée à l'intérieur de la pointe de cylindre (21) et une pointe d'aiguille (3a) se projetant vers un côté d'extrémité distale au-delà de la pointe de cylindre (21), et permettant la communication entre la cavité (22) et un extérieur du cylindre externe (2), l'aiguille d'injection (3) étant fixée à l'intérieur de la pointe de cylindre (21) par moulage par insertion ou thermosoudage,
**caractérisé en ce que**
une partie non homogène (32) ayant une rugosité de surface moyenne d'au moins 0,2 µm est formée dans une région ayant une aire d'au moins 1 mm² sur une surface périphérique externe de la partie articulation (30),
la partie articulation (30) est fermement fixée à l'intérieur de la pointe de cylindre (21) par la résine qui est en contact étroit avec et couvre les renfoncements (32a) et saillies (32b) respectifs de la partie non homogène (32),
le cylindre externe (2) ayant l'aiguille d'injection (3) fixée à l'intérieur de la pointe de cylindre (21) a été stérilisé en autoclave ou par gaz d'oxyde d'éthylène,
et
l'aiguille d'injection (3) comporte une partie extrémité proximale (33) qui est disposée à l'opposé de la pointe d'aiguille (3a) de l'aiguille d'injection (3) et n'est pas dotée de la partie non homogène (32).

2. Seringue (1) avec une aiguille d'injection (3) selon la revendication 1, dans laquelle la partie non homogène (32) a été formée par traitement abrasif.

3. Seringue (1) avec une aiguille d'injection (3) selon la revendication 1, dans laquelle la partie non homogène (32) est formée avec une pluralité de parties non homogènes (32) à un intervalle égal dans une direction circonférentielle de l'aiguille d'injection (3).

4. Seringue (1) avec une aiguille d'injection (3) selon la revendication 1, dans laquelle une longueur de la partie articulation (30) dans une direction axiale de l'aiguille d'injection (3) est d'un maximum de 10 mm.

5. Seringue (1) avec une aiguille d'injection (3) selon la revendication 1, comprenant un capuchon (6) fixé de manière détachable à la pointe de cylindre (21) et configuré pour sceller la pointe d'aiguille (3a).

6. Seringue (1) avec une aiguille d'injection (3) selon la revendication 5, comprenant une solution médicamenteuse (5) remplie dans la cavité (22), et un joint d'étanchéité (4) disposé coulissant dans la section corps (23) de manière étanche au liquide.

7. Procédé de fabrication d'une seringue (1) avec une aiguille d'injection (3) ayant :
un cylindre externe de forme cylindrique (2) moulé avec une résine et comportant une pointe de cylindre (21) au niveau d'une extrémité distale, une ouverture (25) au niveau d'une extrémité proximale, et une section corps (23) comportant une cavité (22) située entre la pointe de cylindre (21) et l'ouverture (25) et configurée pour être remplie d'une solution médicamenteuse (5) ; et
une aiguille d'injection métallique (3) comportant une partie articulation (30) fixée à l'intérieur de la pointe de cylindre (21) et une pointe d'aiguille (3a) se projetant vers un côté d'extrémité distale au-delà de la pointe de cylindre (21), et permettant la communication entre la cavité (22) et un extérieur du cylindre externe (2),
**caractérisé en ce que**
le procédé de fabrication comprend :
la formation d'une partie non homogène (32) ayant une rugosité de surface moyenne d'au moins 0,2 µm dans une région ayant une aire d'au moins 1 mm² sur une surface périphérique externe de la partie articulation (30) ;
le moulage du cylindre externe (2) avec l'aiguille d'injection (3) par réalisation d'un moulage par insertion au moment du moulage du cylindre externe (2) avec la résine, de telle sorte que la partie articulation (30) est fermement fixée à l'intérieur de la pointe de cylindre (21) en raison de la résine qui est en contact étroit avec et couvre les renfoncements (32a) et saillies
(32b) respectifs de la partie non homogène (32), ou le soudage thermique de l'aiguille d'injection (3) au cylindre externe (2) après le moulage du cylindre externe (2) avec la résine, de telle sorte que la partie articulation (30) est fermement fixée à l'intérieur de la pointe de cylindre (21) par la résine qui est en contact étroit avec et couvre les renfoncements (32a) et saillies (32b) respectifs de la partie non homogène (32) ; et
la stérilisation du cylindre externe (2) en utilisant un autoclave ou du gaz d'oxyde d'éthylène,
dans lequel
l'aiguille d'injection (3) comporte une partie extrémité proximale (33) qui est disposée à l'opposé de la pointe d'aiguille (3a) de l'aiguille d'injection (3) et n'est pas dotée de la partie non homogène (32).

8. Procédé de fabrication d'une seringue (1) avec une aiguille d'injection (3) selon la revendication 7, dans lequel la formation de la partie non homogène (32) est réalisée par traitement abrasif.

9. Procédé de fabrication d'une seringue (1) avec une aiguille d'injection (3) selon la revendication 8, dans lequel le traitement abrasif est exécuté par projection de matériau abrasif d'une pluralité de directions à un intervalle égal dans une direction circonférentielle de l'aiguille d'injection (3).
